# EUROPÄISCHE PATENTSCHRIFT

(11) **EP 1 633 294 B1**
(45) Veröffentlichungstag und Bekanntmachung des Hinweises auf die Patenterteilung: **04.03.2009**
(21) Anmeldenummer: 04736961.6
(22) Anmeldetag: 17.06.2004
(51) Int. Cl.: A61F 9/007

(54) **FRÄSWERKZEUG ZUM ABTRAGEN VON EPITHELGEWEBE EINER KORNEA**
MILLING TOOL FOR REMOVING EPITHELIAL TISSUE OF A CORNEA
OUTIL DE FRAISAGE POUR ENLEVER DU TISSU EPITHELIAL DE LA CORNEE

(30) Priorität: 17.06.2003 AT 2003939
(43) Veröffentlichungstag der Anmeldung: 15.03.2006
(73) Patentinhaber: Universa Kunststofftechnik GmbH & Co. KG, 5351 Aigen-Voglhub (AT)
(72) Erfinder: GROSSCHÄDL, Werner, A-4820 Bad Ischl (AT); SECRÉTAN, Michel, CH-1006 Lausanne (CH)
(74) Vertreter: Hübscher, Helmut
(86) Internationale Anmeldenummer: PCT/AT2004/000208
(87) Internationale Veröffentlichungsnummer: WO 2004/110321

(56) Entgegenhaltungen:
- EP-A- 0 458 653
- DE-A- 19 711 675
- GB-A- 2 100 990
- US-A- 5 792 160

## Beschreibung

### Technisches Gebiet

Die Erfindung bezieht sich auf ein Fräswerkzeug zum Abtragen von Epithelgewebe einer Kornea mit einem rotierenden Fräskörper, über dessen Umfangsfläche Spanflächen bildende Schneiden verteilt sind.

### Stand der Technik

Da nach der Entfernung von metallischen Fremdkörpern aus der Kornea mit Korrosionsrückständen in den Wundrändern gerechnet werden muß, wird das Epithelgewebe im Bereich der Wundränder mit Hilfe eines Fräswerkzeuges abgetragen, das einen metallischen, kugelförmigen Fräskörper mit hinterschliffenen Schneiden aufweist. Solche Fräswerkzeuge werden aber auch zum Abtragen angegriffenen Epithelgewebes bei Entzündungen oder anderen Läsionen der Kornea eingesetzt. Um eine Verschleppung von solchen Geweberesten durch die Fräswerkzeuge zu vermeiden, müssen diese Fräswerkzeuge sterilisiert werden, was jedoch schwierig ist, weil feinste Partikel des abgetragenen Gutes am Fräskörper verbleiben und vom Fräskörper wegen der hinterschliffenen Schneiden kaum mechanisch entfernt werden können. Dazu kommt, daß bei Herpeserkrankungen oder ätiologisch unklaren Entzündungen und tumorverdächtigen Läsionen der Kornea Gewebeproben entnommen werden müssen, was den Einsatz gesonderter Instrumente erfordert.

Ähnliche Nachteile ergeben sich bei anderen bekannten Werkzeugen zum Abtragen von Epithelgewebe als Vorbereitung für eine Laserbehandlung der Kornea (WO 99/60963 A1), wenn als Abtragwerkzeug nicht eine rotierende Bürste, sondern ein Rotor aus Metall oder Kunststoff mit einer konkaven Stirnseite eingesetzt wird, auf der radial verlaufende Messer vorgesehen sind, weil auch bei solchen Werkzeugen weder eine Probenentnahme noch eine einfache Sterilisation möglich ist.

### Darstellung der Erfindung

Der Erfindung liegt somit die Aufgabe zugrunde, ein Fräswerkzeug der eingangs geschilderten Art zu schaffen, das nicht nur zum Abtragen von Epithelgewebe, sondern auch zur Probenentnahme geeignet ist und keine Schwierigkeiten hinsichtlich einer Sterilisation macht.

Die Erfindung löst die gestellte Aufgabe dadurch, daß der aus Kunststoff gespritzte Fräskörper eine zylindrische Grundform hat und daß die Spanflächen der Schneiden taschenartige Aussparungen zur Aufnahme von abgetragenem Gewebe aufweisen.

Durch das Vorsehen eines Fräswerkzeuges mit einem in seiner Grundform zylindrischen Fräskörper werden vorteilhafte Voraussetzungen geschaffen, um Epithelgewebe der Kornea nicht nur abzutragen, sondern auch für eine Probenentnahme zu sammeln. Zu diesem Zweck werden in den Spanflächen der Schneiden des Fräskörpers taschenartige Aussparungen vorgesehen, in denen sich abgetragenes Gewebe sammelt, und zwar in einer dem Aufnahmevolumen der taschenartigen Aussparungen entsprechenden Menge, so daß über die Größe dieser Aussparungen die entnommene Probenmenge vorgegeben werden kann. Die an der Spanfläche der Schneiden vorgesehenen Aussparungen beeinflussen den Fräsvorgang selbst nicht. Da der Fräskörper aus Kunststoff gespritzt wird, ist die Ausbildung solcher taschenartiger Aussparungen in den Spanflächen der Schneiden trotz der Kleinheit der Fräskörper in einem wirtschaftlich tragbaren Rahmen möglich. Die Fertigung des Fräskörpers aus Kunststoff erlaubt aber nicht nur eine besondere Ausbildung der Spanflächen der Schneiden mit taschenartigen Aussparungen, sondern stellt eine wesentliche Voraussetzung für einen nur einmaligen Gebrauch des Fräswerkzeuges dar, womit die sonst bei einem Wiedereinsatz von Fräswerkzeugen verbundenen Schwierigkeiten hinsichtlich der Sterilisierung entfallen.

Wie bereits ausgeführt wurde, bestimmt das Volumen der taschenartigen Aussparungen in den Spanflächen der Schneiden, die entnommene Probenmenge, so daß es vielfach ausreichen wird, wenn sich die Aussparungen in den Spanflächen der Schneiden nur über einen Teil der Schneidenlänge erstrecken. Diese Begrenzung der Aussparungen auf Teilabschnitte der Spanflächen bringt Vorteile hinsichtlich der Festigkeit der Schneiden mit sich, insbesondere dann, wenn die Aussparungen mit axialem Abstand vom stirnseitigen Auslauf der Schneiden vorgesehen sind.

### Kurze Beschreibung der Zeichnung

In der Zeichnung ist der Erfindungsgegenstand beispielsweise dargestellt. Es zeigen:

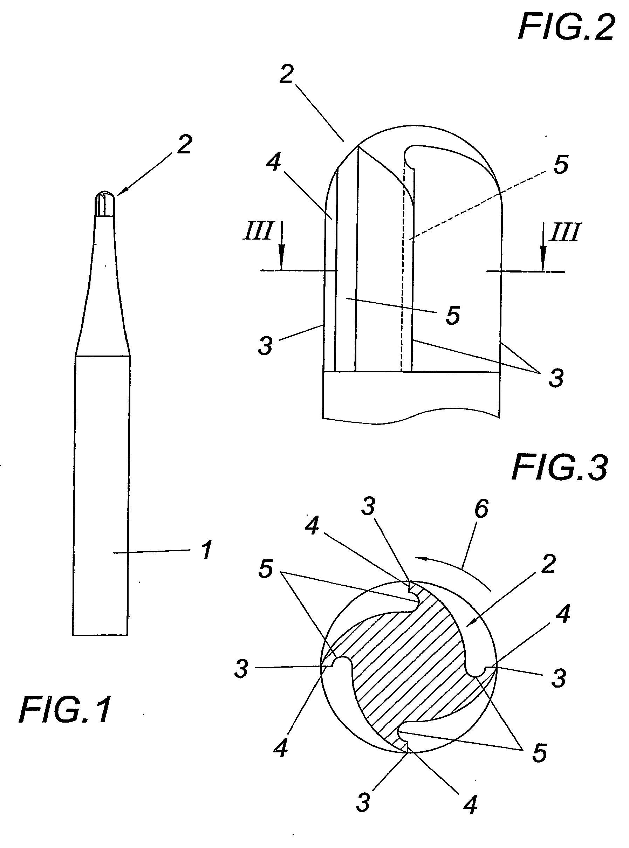

- Fig. 1: ein erfindungsgemäßes Fräswerkzeug zum Abtragen von Epithelgewebe einer Kornea in einer Seitenansicht,
- Fig.: 2 den Fräskörper dieses Fräswerkzeuges in einer Seitenansicht in einem größeren Maßstab,
- Fig.: 3 einen Schnitt nach der Linie III-III der Fig. 2,
- Fig.: 4 eine Ausführungsvariante eines erfindungsgemäßen Fräskörpers in einer zum Teil aufgerissenen Seitenansicht in einem größeren Maßstab und

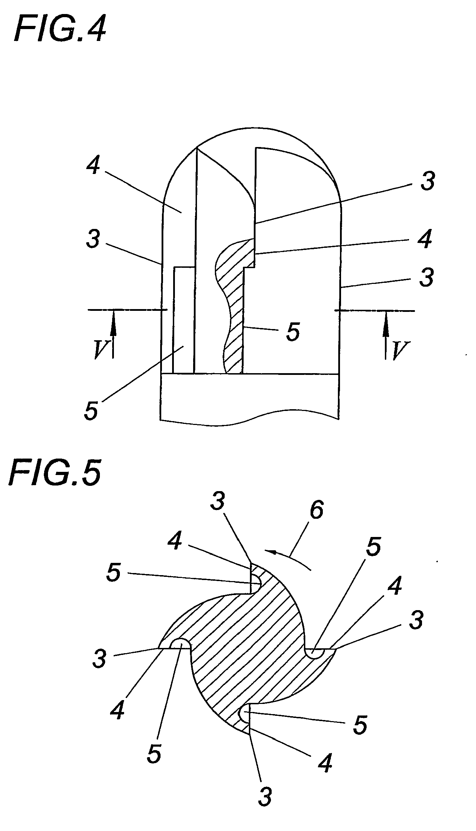

- Fig. 5: einen Schnitt nach der Linie V-V der Fig. 4.

### Weg zur Ausführung der Erfindung

Das Fräswerkzeug gemäß der Fig. 1 weist einen in ein Spannfutter eines elektrischen Drehantriebes einsetzbaren Schaft 1 auf, der an seinem verjüngten, freien Ende den Fräskörper 2 trägt. Dieser Fräskörper 2 hat eine zylindrische Grundform und bildet über den Umfang verteilte, axial verlaufende Schneiden 3, deren Spanflächen mit 4 bezeichnet sind. In diesen Spanflächen 4 sind taschenartige Aussparungen 5 vorgesehen, die gemäß den Fig. 2 und 3 über die Schneidenlänge durchlaufen.

Beim Einsatz des Fräswerkzeuges wird durch die Schneiden 3 des sich in Richtung des Pfeiles 6 drehenden Fräskörpers 2 Epithelgewebe von der Kornea abgetragen, das sich in den taschenartigen Aussparungen 5 der Spanfläche 4 sammelt, bis die Aussparungen 5 gefüllt sind. Nach dem Fräsvorgang können die abgetragenen Gewebeteile aus den Aussparungen 5 entnommen und beispielsweise einer bakteriologischen Untersuchung unterworfen werden. Um die Entnahme von Gewebeproben aus der Epithelschicht der Kornea zu erleichtern, kann das Fräswerkzeug unter Umständen mit einer geringeren Umlaufgeschwindigkeit angetrieben werden.

Zum Unterschied zur Ausbildungsform der Fig. 2 und 3 erstrecken sich die taschenartigen Aussparungen 5 in den Spanflächen 4 der Schneiden 3 gemäß der Konstruktionsvariante des in den Fig. 4 und 5 dargestellten Fräskörpers 2 nur über eine Teillänge der Schneiden 3. Die Anordnung ist dabei so getroffen, daß die Aussparungen 5 mit axialem Abstand vom stirnseitigen Auslauf der Schneiden 3 vorgesehen sind, wie dies insbesondere der Fig. 4 entnommen werden kann. Diese in der axialen Erstreckung des Fräskörpers 2 beschränkte Länge der Aussparungen 5 bringt im Bereich der freien Stirnseite des Fräskörpers 2 eine höhere Festigkeit für die Schneiden 3 mit sich, ohne eine entsprechende Probenentnahme zu gefährden.

## Patentansprüche

1. Fräswerkzeug zum Abtragen von Epithelgewebe einer Kornea mit einem rotierenden Fräskörper, über dessen Umfangsfläche Spanflächen bildende Schneiden verteilt sind, **dadurch gekennzeichnet, daß** der aus Kunststoff gespritzte Fräskörper (2) eine zylindrische Grundform hat und daß die Spanflächen (4) der Schneiden (3) taschenartige Aussparungen (5) zur Aufnahme von abgetragenem Gewebe aufweisen.

2. Fräswerkzeug nach Anspruch 1, **dadurch gekennzeichnet, daß** sich die Aussparungen (5) in den Spanflächen (4) der Schneiden (3) nur über einen Teil der Schneidenlänge erstrecken.

3. Fräswerkzeug nach Anspruch 2, **dadurch gekennzeichnet, daß** die Aussparungen (5) mit axialem Abstand vom stirnseitigen Auslauf der Schneiden (3) vorgesehen sind.

## Claims

1. Milling tool for removing epithelial tissue of a cornea, having a rotating milling body, wherein cutters Forming cutting surfaces are distributed over the peripheral surface of the milling body, **characterised in that** the milling body (2) extruded from synthetic material has a cylindrical basic shape, and **in that** the cutting surfaces (4) of the cutters (3) comprise pocket-like recesses (5) for receiving removed tissue.

2. Milling tool as claimed in Claim 1, **characterised in that** the recesses (5) extend in the cutting surfaces (4) of the cutters (3) only over a portion of the length of the cutters.

3. Milling tool as claimed in Claim 2, **characterised in that** the recesses (5) are provided at an axial spaced disposition with respect to the end-side projection of the cutters (3).

## Revendications

1. Outil de fraisage pour enlever du tissu épithélial d'une cornée, avec un corps de fraise rotatif sur la surface périphérique duquel sont répartis des dents formant des surfaces à copeaux, **caractérisé en ce que** le corps de fraise (2), obtenu par injection de matière synthétique, présente une forme de base cylindrique, et **en ce que** les surfaces à copeaux (4) des dents (3) présentent des évidements (5) du genre de poches, pour recevoir du tissu ayant été enlevé.

2. Outil de fraisage selon la revendication 1, **caractérisé en ce que** les évidements (5) ménagés dans les surfaces à copeaux (4) des dents (3) ne s'étendent que sur une partie de la longueur de dent.

3. Outil de fraisage selon la revendication 2, **caractérisé en ce que** les évidements (5) sont prévus selon un espacement axial vis-à-vis de l'extrémité frontale des dents (3) .
